# EUROPÄISCHE PATENTANMELDUNG

(11) **EP 1 050 269 A1**
(43) Veröffentlichungstag der Anmeldung: **08.11.2000**
(21) Anmeldenummer: 99890149.0
(22) Anmeldetag: 07.05.1999
(51) Int. Cl.: A61B 5/0408

(54) **Mehrkontakt-Elektrode**

(71) Anmelder: Nessler Medizintechnik GmbH & Co KG, 6060 Absam (AT)
(72) Erfinder: Nessler, Winfried, Dr., 6020 Innsbruck (AT)
(74) Vertreter: Häupl, Armin, Dipl.-Ing.

(57) **Zusammenfassung**

Die Erfindung offenbart eine neue Mehrkontakt-Elektrode, insbesondere zur Verwendung für EKG-Untersuchungen, umfassend: mehrere voneinander elektrisch isolierte Kontaktzonen (1) zur Herstellung von Kontakt mit der Haut, aus elektrisch leitendem Material mit definierter Größe, geometrischer Anordnung und zugehörigen, damit einstückig ausgebildeten Leiterbahnen (2), die voneinander und von der Haut elektrisch isoliert sind, zum Verbinden der Kontaktzonen (1) mit Zu- oder Ableitungen, insbesondere einem alle Leiterbahnen (2) an deren Anschlußenden (3) kontaktierenden Mehrkontaktstecker; eine auf der Oberfläche der Kontaktzonen (1) vorgesehene elektrisch leitende Kleberschicht (4) zur Herstellung von elektrischem Kontakt zwischen der Haut und den Kontaktzonen (1); eine auf der der Haut zugewandten Seite der Leiterbahnen (2) unter Aussparung der Anschlußenden (3) vorgesehene und die Leiterbahnen (2) von der Haut elektrisch isolierende, beidseitig klebende Folie (5); und eine stabilisierende, an der den Kontaktzonen und der Haut zugewandten Seite klebende Träger-Deckschicht (6).

## Beschreibung

Die vorliegende Erfindung betrifft Mehrkontakt-Elektroden, insbesondere Mehrkontakt-Elektroden zur Verwendung für EKG-Untersuchungen, sowie Verfahren zu deren Herstellung.

Zahlreiche in der modernen Medizin angewandte Untersuchungs- und Behandlungsmethoden basieren auf direktem elektrischem Kontakt zwischen dem Körper des Patienten und darauf angebrachten Elektroden, wie z.B. zur Aufnahme von EKG (Elektrokardiogramm), EEG (Elektroenzephalogramm) und EMG (Elektromyogramm) sowie für therapeutische Anwendungen wie TENS (transkutane elektrische Nervenstimulation) und EMS (elektrische Muskelstimulation).

Um dem Körper des Patienten zu diesen Zwecken elektrischen Strom zuführen oder elektrische Signale vom Körper erhalten zu können, werden in der Regel mehrere Elektroden am Körper haftend angebracht, von denen manche zur Mehrfachbestimmung desselben Signals dienen können, während manchen anderen unterschiedliche Aufgaben zukommen, deren Anordnung und elektrische Isolation voneinander allerdings in jedem Fall für den Erfolg der Messung oder Behandlung ausschlaggebend ist.

Beispielsweise werden beim EKG 6 Kontakte im Brustbereich benötigt, bei manchen neuen EKG-Methoden sogar bis zu 100. Jeden Kontakt einzeln, d.h. über herkömmliche Ein-Kontakt-EKG-Elektroden, herzustellen und dann von jeder Elektrode ein eigenes einpoliges Kabel zu einem Rekorder zu führen, ist für den Anwender äußerst aufwendig.

In der HF-(Hochfrequenz-)Chirurgie werden gegenwärtig Zwei- und bisweilen auch Drei-Kontakt-Neutralelektroden eingesetzt, um eine automatische Kontaktüberwachung des HF-Generators zu ermöglichen (d.h. der Rückstrom muß über alle Teilkontakte gleich groß sein). Die elektrische Verbindung der Konzaktzonen solcher HF-Elektroden mit den Ableitkabeln erfolgt allerdings über leitende Drahtverbindungen (z.B. aus Edelmetallen), was nicht nur kostenintensiv ist sondern auch keine besonders stabile Art der Verbindung darstellt (die Leiterfolie wird vom Drahtverbinder durchdrungen, der jedoch leicht ausreißt).

Es besteht daher ein allgemeiner Bedarf an kostengünstig herstellbaren, mit beschädigungssicheren Anschluß-Verbindungen versehenen Mehrkontakt-Elektroden für sämtliche Bereiche der modernen Elektromedizin, insbesondere aber für EKG-Messungen, wo die Anzahl der erforderlichen Elektroden besonders hoch ist.

Dieses Ziel wird durch die vorliegende Erfindung in ihrem ersten Aspekt durch Bereitstellung einer Mehrkontakt-Elektrode erreicht, die sich insbesondere für EKG-Untersuchungen eignet und folgendes umfaßt:
mehrere voneinander elektrisch isolierte Kontaktzonen zur Herstellung von Kontakt mit der Haut, aus elektrisch leitendem Material mit definierter Größe, geometrischer Anordnung und zugehörigen, damit einstückig ausgebildeten Leiterbahnen, die voneinander und von der Haut elektrisch isoliert sind, zum Verbinden der Kontaktzonen mit Zu- oder Ableitungen, insbesondere einem alle Leiterbahnen an deren Anschlußenden kontaktierenden Mehrkontaktstecker;
eine auf der Oberfläche der Kontaktzonen vorgesehene elektrisch leitende Kleberschicht zur Herstellung von elektrischem Kontakt zwischen der Haut und den Kontaktzonen;
eine auf der der Haut zugewandten Seite der Leiterbahnen unter Aussparung der Anschlußenden vorgesehene und die Leiterbahnen von der Haut elektrisch isolierende, beidseitig klebende Folie; und
eine stabilisierende, an der den Kontaktzonen und der Haut zugewandten Seite klebende Träger-Deckschicht.

Bei einer solchen Elektrode ist aufgrund der elektrischen Verbindung zwischen Kontaktzone und Stecker über einstückig mit der Kontaktzone ausgebildete Leiterbahnen und Anschlußenden hohe Sicherheit gegen Ausreißen der Leiterbahnen gegeben, und es erübrigt sich der Einsatz teurer Metall-Teile, wodurch die Kosten reduziert werden können.

In einer besonders bevorzugten Ausführungsform weisen die Kontaktzonen, Leiterbahnen und Anschlußenden eine derartige Größe und geometrische Anordnung auf, daß sie aus einer elektrisch leitfähigen Folie in einem einzigen Schneid- oder Stanzschritt herstellbar sind, wodurch die erfindungsgemäßen Elektroden vollautomatisiert und damit kostengünstig herstellbar sind.

Die Anzahl der Kontaktzonen beträgt vorzugsweise zumindest 6, wodurch die erfindungsgemäßen Elektroden für EKG-Messungen bestens geeignet ist.

Das Material für die Kontaktzonen, Leiterbahnen und Anschlußenden ist dabei vorzugsweise eine flexible, elektrisch leitfähige Folie, insbesondere Metallfolien wie Al-Folien, Al-Verbundfolien oder ein Gewebe aus elektrisch leitfähigen Fasern, wodurch die Elektroden -- neben der geringen Materialkosten - gut an die Körperkontouren des Patienten anpaßbar sind. Zu diesen Zwecken weist die Folie vorzugsweise eine Dicke von nicht mehr als 2 mm, besonders bevorzugt nicht mehr als 1 mm, insbesondere unter 0,1 mm, auf.

Die elektrisch leitende Kleberschicht kann dabei in herkömmlicher Weise aus elektrisch leitfähigen Klebern und Hydrogelen ausgewählt sein, die eine Dicke von nicht mehr als 2 mm, vorzugsweise nicht mehr als 1 mm, insbesondere unter 0,5 mm, aufweisen, um die Kosten bei guter elektrischer Leitfähigkeit und Haftkraft niedrig zu halten.

Es kann weiters eine von bisherigen Elektroden bekannte Träger-Deckschicht aus Schaumstoff oder Pflastermaterialien eingesetzt werden, deren Dicke üblicherweise im Bereich unter 0,5 mm, vorzugsweise 80-250 µm, liegt, was einen guten Kompromiß zwischen der Stabilität der Elektrode einerseits und ihrer Flexibilität sowie den Herstellungskosten andererseits bietet.

In einem zweiten Aspekt umfaßt die vorliegende Erfindung ein Verfahren zur Herstellung von Mehrkontakt-Elektroden wie oben beschrieben, welches durch folgende Schritte gekennzeichnet ist:
zumindest teilweises Beschichten einer elektrisch leitfähigen Folie, vorzugsweise Al-Folie, mit einer elektrisch leitenden Kleberschicht, vorzugsweise einem elektrisch leitfähigen Hydrogel;
gleichzeitiges Ausschneiden bzw. -stanzen von Kontaktzonen, Leiterbahnen und Anschlußenden aus der mit Kleber bzw. Hydrogel beschichteten Folie;
Aufbringen, vorzugsweise Aufkaschieren, einer durchwegs klebenden Träger-Deckschicht, vorzugsweise aus Schaumstoff; und
Abdecken der Leiterbahnen mit einer isolierenden, beidseitig klebenden Folie.

Ein solches Verfahren zeichnet sich durch einen extrem einfachen Herstellungsschritt der elektrisch leitenden Komponenten durch einfaches Ausschneiden bzw. -stanzen aus und kann vollautomatisiert durchgeführt werden, was einen hohen Output an Elektroden und daher eine rasche und billige Variante der Herstellung solcher Elektroden ermöglicht.

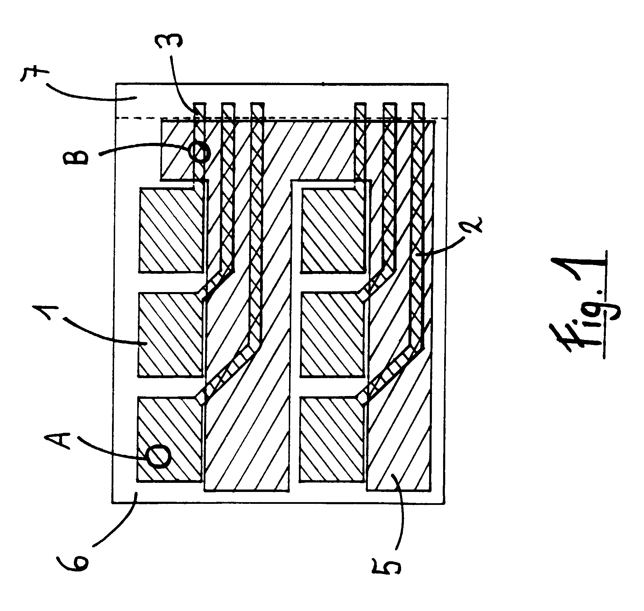

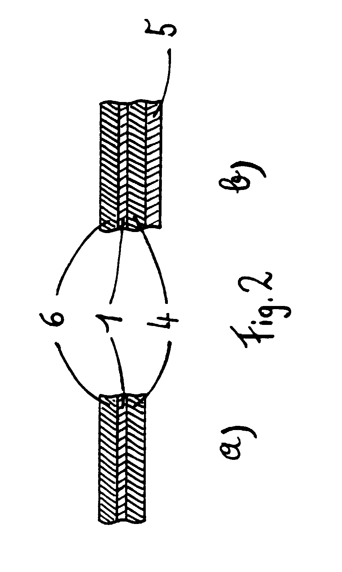

Die vorliegende Erfindung wird nachstehend unter Bezugnahme auf die beiligenden Zeichnungen näher beschrieben, worin Fig. 1 eine schematische Darstellung einer erfindungsgemäßen Mehrkontakt-Elektrode mit 6 Kontaktzonen von unten ist und die Fig. 2a) und 2b) schematische Querschnittsansichten der mit A und B gekennzeichneten Bereiche der Elektrode aus Fig. 1 sind.

In Fig. 1 sind die einzelnen leitenden Teile einer erfindungsgemäßen Elektrode in einer schematischen Unteransicht der Elektrode dargestellt. Auf einem durchwegs klebenden Träger 6, z.B. aus geschäumtem Kunststoff, sind sechs Kontaktzonen 1 mit zugehörigen, einstückig ausgebildeten Leiterbahnen 2 zu erkennen, wobei letztere die Kontaktzonen 1 mit einer (in der Abbildung durch eine strichlierte Linie begrenzte) Anschlußzone (z.B. Steckerleiste) 7 verbinden. Die Leiterbahnen 2 sind mit einer beidseitig klebenden nichtleitenden Folie 5 zur Haut hin abgedeckt und voneinander elektrisch isoliert, wobei allerdings die Anschlußenden 3, die zum Verbinden der Leiterbahnen 2 mit Zu- oder Ableitungen, insbesondere einem (nicht dargestellten) alle Anschlußenden 3 kontaktierenden Mehrkontaktstecker, dienen, im Bereich der Anschlußzone 7 freiliegen und gegebenenfalls nicht mit der leitfähigen Kleberschicht beschichtet sind.

Die Kontaktzonen 1 und die Leiterbahnen 2 mit ihren Anschlußenden 3 sind dabei aus einer mit einer elektrisch leitfähigen Kleberschicht 4 (z.B. leitfähiger Kleber oder Hydrogel) überzogenen, elektrisch leitenden Folie, z.B. Metallfolie aus Aluminium oder Al-Verbundfolie oder dergleichen, einstückig ausgestanzt oder-geschnitten, was die Stabilität der Leiteranordnung gegenüber herkömmlichen Drahtverbindungen beträchtlich erhöht.

Die Anschlußzone 7 ist zwar in Fig. 1 als einfaches Rechteck dargestellt, kann aber dem jeweiligen Mehrkontakt-Stecker entsprechend, z.B. symmetrisch, abgerundet oder dergleichen, geformt sein.

In Fig. 2 sind schematische Querschnitte durch die Elektrode aus Fig. 1 zu erkennen, wobei Fig. 2a) einen Querschnitt an Position A aus Fig. 1, d.h. im Bereich der Kontaktzonen 1, und Fig. 2b) einen Querschnitt an Position B aus Fig. 1, d.h. im Bereich der Leiterbahnen 2, zeigt. Von oben nach unten (d.h. zur Haut des Patienten hin) sind in beiden Fällen der an der Unterseite klebende Träger 6, die elektrisch leitende Folie der Kontaktzonen 1 und die elektrisch leitende Kleberschicht 4, die bei Verwendung der Elektrode mit der Haut in Kontakt steht, dargestellt. In Fig. 2b) ist zudem als unterste, ebenfalls mit der Haut in Kontakt befindliche Schicht die elektrisch nichtleitende, beidseitig klebende Folie 5 gezeigt, die die Leiterbahnen von der Haut elektrisch isoliert.

Die Dicke der einzelnen Schichten ist in der Figur zur Illustration willkürlich angenommen, beträgt aber im einzelnen vorzugsweise unter 1 mm, insbesondere unter 0,1 mm, z.B. 50 µm, für die leitende Folie 1, unter 2 mm, insbesondere unter 1 mm, z.B. 0,3 mm, für die leitende Kleberschicht 4, sowie unter 0,5 mm, z.B. 160 µm, für die Trägerschicht 6. Die Dicke der Isolierfolie 5 ist nicht speziell eingeschränkt und kann möglichst dünn, jedoch ausreichend dick sein, um die Leiterbahnen zur Gänze voneinander und von der Haut zu isolieren, z.B. 50 bis 100 µm.

Die in Fig. 1 dargestellte Mehrkontakt-Elektrode ist insbesondere für EKG-Untersuchungen ausgezeichnet geeignet, kann aber in dieser oder ähnlicher Form auch für sämtliche anderen elektromedizinischen Anwendungen adaptiert werden.

Die Herstellung der erfindungsgemäßen Elektroden, wie in den Figuren dargestellt, erfolgt geeigneterweise nach dem erfindungsgemäßen Verfahren als zweiter Aspekt der Erfindung. Dabei wird zunächst eine elektrisch leitfähige Folie, vorzugsweise Al-Folie oder Al-Verbundfolie, mit der elektrisch leitenden Kleberschicht 4, vorzugsweise einem elektrisch leitfähigen Hydrogel, beschichtet. Die Beschichtung kann nach jedem geeigneten Verfahren erfolgen, z.B. Rakelbeschichtung, Spritzbeschichtung oder dergleichen.

Anschließend werden gleichzeitig, also in einem Verfahrensschritt, die Kontaktzonen 1 und die Leiterbahnen 2 (mit ihren Anschlußenden 3) aus der mit Kleber bzw. Hydrogel beschichteten Folie ausgeschnitten oder -gestanzt, wobei bereits die spätere geometrische Anordnung der Kontaktzonen 1 und Leiterbahnen 2 erhalten wird.

Danach wird auf die Oberseite der Anordnung eine durchwegs klebende Träger-Deckschicht 6, z.B. aus Schaumstoff bzw. Pflastermaterialien, vorzugsweise durch Aufkaschieren, aufgebracht, und zuletzt werden die Leiterbahnen 2 mit einer isolierenden, beidseitig klebenden Folie 5 abgedeckt, wobei die Anschlußenden 3 ausgespart werden, um eine Anschlußzone 7 zu bilden, was die fertige Elektrode ergibt.

Als letzter Schritt wird üblicherweise auf der durchwegs klebenden Unterseite der Elektrode eine (nicht dargestellte) Abziehfolie vorgesehen, um die Elektrode während der Lagerung und des Hantierens gegen Beeinträchtigungen zu schützen. Diese Abziehfolie wir erst unmittelbar vor Verwendung der Elektrode entfernt und dabei die Hautkontakt-Flächen freigelegt.

## Patentansprüche

1. Mehrkontakt-Elektrode, insbesondere zur Verwendung für EKG-Untersuchungen, die umfaßt:
mehrere voneinander elektrisch isolierte Kontaktzonen (1) zur Herstellung von Kontakt mit der Haut, aus elektrisch leitendem Material mit definierter Größe, geometrischer Anordnung und zugehörigen, damit einstückig ausgebildeten Leiterbahnen (2), die voneinander und von der Haut elektrisch isoliert sind, zum Verbinden der Kontaktzonen (1) mit Zu- oder Ableitungen, insbesondere einem alle Leiterbahnen (2) an deren Anschlußenden (3) kontaktierenden Mehrkontaktstecker;
eine auf der Oberfläche der Kontaktzonen (1) vorgesehene elektrisch leitende Kleberschicht (4) zur Herstellung von elektrischem Kontakt zwischen der Haut und den Kontaktzonen (1);
eine auf der der Haut zugewandten Seite der Leiterbahnen (2) unter Aussparung der Anschlußenden (3) vorgesehene und die Leiterbahnen (2) von der Haut elektrisch isolierende, beidseitig klebende Folie (5); und
eine stabilisierende, an der den Kontaktzonen und der Haut zugewandten Seite klebende Träger-Deckschicht (6).

2. Elektrode nach Anspruch 1, dadurch gekennzeichnet, daß die Kontaktzonen (1), Leiterbahnen (2) und Anschlußenden (3) eine derartige Größe und geometrische Anordnung aufweisen, daß sie aus einer elektrisch leitfähigen Folie in einem einzigen Schneid- oder Stanzschritt herstellbar sind.

3. Elektrode nach Anspruch 1 oder 2, dadurch gekennzeichnet, daß die Anzahl der Kontaktzonen (1 ) zumindest 6 beträgt.

4. Elektrode nach einem der Ansprüche 1 bis 3, dadurch gekennzeichnet, daß Kontaktzonen (1), Leiterbahnen (2) und Anschlußenden (3) aus einer flexiblen, elektrisch leitfähigen Folie bestehen.

5. Elektrode nach Anspruch 4, dadurch gekennzeichnet, daß die Folie aus Metallfolien, vorzugsweise Al-Folien oder Al-Verbundfolien, und Geweben aus elektrisch leitfähigen Fasern ausgewählt ist.

6. Elektrode nach einem der Ansprüche 3 bis 5, dadurch gekennzeichnet, daß die Folie eine Dicke von nicht mehr als 2 mm, vorzugsweise nicht mehr als 1 mm, insbesondere unter 0,1 mm, aufweist.

7. Elektrode nach einem der vorangegangenen Ansprüche, dadurch gekennzeichnet, daß die elektrisch leitende Kleberschicht (4) aus elektrisch leitfähigen Klebern und Hydrogelen ausgewählt ist.

8. Elektrode nach Anspruch 7, dadurch gekennzeichnet, daß die Kleberschicht (4) eine Dicke von nicht mehr als 2 mm, vorzugsweise nicht mehr als 1 mm, insbesondere unter 0,5 mm, aufweist.

9. Elektrode nach einem der vorangegangenen Ansprüche, dadurch gekennzeichnet, daß die Träger-Deckschicht (6) aus Schaumstoff bzw. Pflastermaterialien besteht.

10. Elektrode nach Anspruch 9, dadurch gekennzeichnet, daß die Dicke des Schaumstoffs bzw. der Pflastermaterialien im Bereich von unter 0,5 mm, vorzugsweise 80-250 µm, liegt.

11. Verfahren zur Herstellung einer Mehrkontakt-Elektrode nach einem der Ansprüche 1 bis 10, gekennzeichnet durch folgende Schritte:
zumindest teilweises Beschichten einer elektrisch leitfähigen Folie, vorzugsweise Al-Folie, mit einer elektrisch leitenden Kleberschicht, vorzugsweise einem elektrisch leitfähigen Hydrogel, (4);
gleichzeitiges Ausschneiden bzw. -stanzen von Kontaktzonen (1), Leiterbahnen (2) und Anschlußenden (3) aus der mit Kleber bzw. Hydrogel beschichteten Folie;
Aufbringen, vorzugsweise Aufkaschieren, einer durchwegs klebenden Träger-Deckschicht (6), vorzugsweise aus Schaumstoff; und
Abdecken der Leiterbahnen (2) mit einer isolierenden, beidseitig klebenden Folie (5).
